(19) Europäisches Patentamt / European Patent Office / Office européen des brevets

(11) **EP 2 552 401 B1**

(12) **FASCICULE DE BREVET EUROPEEN**

(45) Date de publication et mention de la délivrance du brevet:
**23.08.2017 Bulletin 2017/34**

(51) Int Cl.:
*A61K 8/97* *(2017.01)*     *A61Q 19/00* *(2006.01)*
*A61Q 19/08* *(2006.01)*     *A61K 8/96* *(2006.01)*

(21) Numéro de dépôt: **11711557.6**

(22) Date de dépôt: **30.03.2011**

(86) Numéro de dépôt international:
**PCT/EP2011/054965**

(87) Numéro de publication internationale:
**WO 2011/121051 (06.10.2011 Gazette 2011/40)**

(54) **UTILISATION D'UNE PRÉPARATION ISSUE D'UNE CULTURE IN VITRO DE CELLULES DÉDIFFÉRENCIÉES NON ÉLICITÉES D'ARGANIER DANS LE TRAITEMENT DU VIEILLISSEMENT CUTANÉ**

VERWENDUNG EINER ZUBEREITUNG AUS EINER IN-VITRO-KULTUR AUS ENTDIFFERENZIERTEN NICHTSTIMULIERTEN ZELLEN AUS DEM ARGANBAUM ZUR BEHANDLUNG VON HAUTALTERUNG

USE OF A PREPARATION OBTAINED FROM AN IN VITRO CULTURE OF DEDIFFERENTIATED, NON-ELICITED CELLS OF THE ARGANIA TREE FOR TREATING SKIN AGEING

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **31.03.2010 FR 1001327**

(43) Date de publication de la demande:
**06.02.2013 Bulletin 2013/06**

(73) Titulaire: **Pierre Fabre Dermo-Cosmétique**
**92100 Boulogne-Billancourt (FR)**

(72) Inventeurs:
• **STEWARD, Nicolas**
  **F-31860 Pins-Justaret (FR)**
• **MANDEAU, Anne**
  **F-31200 Toulouse (FR)**
• **CASTEX-RIZZI, Nathalie**
  **F-31770 Colomiers (FR)**

(74) Mandataire: **Regimbeau**
**20, rue de Chazelles**
**75847 Paris Cedex 17 (FR)**

(56) Documents cités:
**EP-A1- 1 213 025     WO-A1-03/077880**
**FR-A1- 2 847 476**

EP 2 552 401 B1

**Description**

La présente invention a pour objet une préparation issue d'une culture *in vitro* de cellules dédifférenciées non élicitées d'Arganier, une composition cosmétique ou dermatologique comprenant ladite préparation et ses utilisations pour le traitement du vieillissement cutané, de l'inflammation et de la cicatrisation l'utilisation d'une préparation issue d'une culture in vitro de cellules dédifférenciées non élicitées d'Arganier ou d'une composition cosmétique la contenant, dans le traitement non thérapeutique du vieillissement cutané. L'Arganier est un arbre qui appartient à la famille botanique des Sapotacées et porte le nom scientifique *Argania spinosa* (L.) Scelles.

Son port rappelle celui de l'Olivier, son tronc est court, tortueux. Le bois est très dur et dense. Les rameaux sont très épineux et portent des feuilles petites, lancéolées, alternes, étroites, courtes (env. 2 cm) et souvent regroupées en fascicule. Les feuilles sont persistantes mais, lors de grande sécheresse, elles deviennent caduques et tombent. Les fleurs sont hermaphrodites et pentamères. Elles sont regroupées en glomérule, s'épanouissent en mai juin. Elles sont de couleur jaune-verdâtre.

L'Arganier fructifie dés l'âge de 5 ans. Le fruit est une baie sessile, jaune, ovale, de 4 à 5 cm de long. Elle est constituée d'une pulpe renfermant un faux noyau constitué de 2 à 3 graines aplaties soudées entre elles et renfermant chacune une amande oléagineuse.

L'Arganier est endémique du Maroc et situé principalement dans le sud ouest marocain entre Essaouira et Agadir. L'arganeraie s'étend sur environ 830 000 ha.

Les populations marocaines ont tout d'abord exploité l'Arganier pour son bois particulièrement dur comme fourniture de combustible. L'autre grande utilisation traditionnelle est l'huile extraite dans un premier temps manuellement mais aujourd'hui principalement à l'aide de presse. La première utilisation de cette huile est alimentaire ; aujourd'hui, elle connaît une exploitation importante en cosmétique. Les pulpes et les tourteaux résiduels issus de cette production d'huile sont donnés normalement en alimentation à des animaux.

La cosmétologie a développé de nombreux produits à partir de l'Arganier. L'huile issue des graines a fait l'objet de plusieurs brevets d'invention : obtention de l'huile par solvant (Brevet Fr 2 553 788), l'huile d'Argan enrichie en insaponifiable (Brevet Fr 2 724 663).

Des substances autres que l'huile ont également été brevetées. C'est le cas de peptides issus des tourteaux des graines obtenus après extraction de l'huile : association de l'huile et de peptides de tourteaux pour le traitement des troubles liés au vieillissement cutané (Brevet Fr 2 756 183). La feuille de l'Arganier, les protéines et les saponines de tourteaux ont également fait l'objet de brevets d'invention = Extraits de feuilles (Brevet EP 1 213 025), protéines de Tourteaux (Brevet EP 1 213 024), saponines de Tourteaux (Brevet EP 1 430 900). Plus récemment, la demande de brevet EP 1 968 536 concernant l'utilisation en cosmétique anti-âge d'un extrait réalisé à partir des pulpes de fruit d'Arganier a été déposée.

L'Arganier dans son ensemble présente donc une composition d'intérêt pour un usage dermatologique et/ou cosmétique. L'Arganier est une plante ressource importante, d'un point de vue écologique tout d'abord car c'est une plante « écosystème ». Parfaitement adapté à l'aridité, il protège les sols des érosions hydriques et éoliennes et s'oppose ainsi à l'avancée du désert au Maroc. Mais également d'un point de vue économique, car c'est un arbre à multiples usages.

C'est la plante phare du Maroc. Ainsi, un dahir (décret) daté de 1925 protège l'arganeraie, posant le principe du droit supérieur de l'Etat sur la forêt d'arganiers, mais l'usufruit (fruit, bois morts, cultures sous arganiers...) est laissé à la population locale. Depuis peu, l'arganeraie a été classée réserve de la biosphère par l'UNESCO.

C'est pourquoi l'utilisation de son bois ou de ses feuilles pour un usage cosmétique pourrait avoir des conséquences graves sur cette plante protégée.

Un autre moyen d'obtenir des molécules d'intérêt à partir d'une plante est la préparation de cultures de cellules dédifférenciées totipotentes. L'utilisation de cellules végétales dédifférenciées permet de plus d'éviter certains problèmes d'industrialisation rencontrés lors du développement de produits cosmétiques. En effet, avec la culture cellulaire, la différence de concentration en substances d'intérêts qui existe entre les différents lots ou récoltes de la plante disparaît. C'est également une technique non destructive et sa mise en place est relativement simple et peu coûteuse. Enfin, elle s'affranchit de l'extraction, les cellules étant dans un milieu de culture, et les composés actifs se retrouvant soit dans le milieu de culture, soit dans le liquide intracellulaire. Un simple broyage permet donc de rendre disponibles ces composés. Le document EP 1 213 025 décrit l'utilisation d'un extrait de feuilles d'Arganier dans le traitement des signes du vieillissement cutané. La demande de brevet WO03077881 décrit une composition pour application topique contenant au moins un broyat de cellules végétales dédifférenciées et élicitées en culture in vitro pour synthétiser au moins une phytoalexine. Le matériel végétal est préférentiellement issu de la vigne.

Ainsi ce document enseigne la valorisation cosmétique des cellules végétales susceptibles d'être dédifférenciées et d'être élicitées, l'élicitation menant à l'accumulation de métabolites secondaires en quantité suffisante pour permettre l'activité biologique en usage topique.

Or de façon surprenante et inattendue, la Demanderesse a mis en évidence qu'une préparation, issue d'une culture *in vitro* de cellules dédifférenciées mais non élicitées d'Arganier, présente des bonnes activités cosmétiques et/ou dermatologiques dans le domaine de l'anti-âge. Les résultats obtenus démontrent que, dans le cas spécifique de l'Arganier, une autre valorisation de cultures cellulaires d'Arganier, ici non élicitées, est possible dans les axes cités précédemment.

De plus, l'obtention de la préparation au sens de la présente invention permet de s'affranchir de l'étape d'élicitation, étape lourde et coûteuse d'un point de vue industriel ; l'élicitation entraînant un rendement en biomasse bien inférieur, dû à un ralentissement de la croissance cellulaire.

La demande WO03077881 cite diverses manières de mener cette élicitation, par exemple rayon UV pendant 3 jours ; gaz carbonique pendant 24 heures à 2 jours ; rayon UV et gaz carbonique pendant 5 jours.

Le procédé mené dans le cadre de la présente invention consistant en la dédifférenciation cellulaire à partir de matériel végétal issu d'arganier, puis en la culture de cellules en suspension ; permet d'obtenir rapidement une biomasse fine, abondante, homogène et stérile de cette plante. La culture cellulaire rend manipulable les voies de biosynthèse de cette plante directement à l'échelle cellulaire.

La présente invention vise donc l'utilisation d'une préparation issue d'une culture *in vitro* de cellules dédifférenciées non élicitées d'Arganier, et d'une composition cosmétique ou dermatologique comprenant ladite préparation pour le traitement non thérapeutique du vieillissement cutané. D'une manière plus générale les cultures *in vitro* de tissus végétaux en suspension permettent de produire des composés organiques actifs directement issus du métabolisme primaire ou secondaire des cellules.

Les cellules végétales en suspension sont dans un état de dédifférenciation similaire à celui des cellules souches pour les cultures de cellules animales. Ces cellules végétales sont donc théoriquement capables de produire tous les métabolites observés dans la plante entière. La dédifférenciation entraîne une perturbation des voies de biosynthèse d'ordre génétique ou épigénétique si bien que les profils chimiques sont différents quantitativement et qualitativement entre la plante entière et les souches cellulaires qui en découlent. Ainsi théoriquement des intermédiaires réactionnels non observés dans la plante entière peuvent apparaître en suspension cellulaire. Ceci offre une nouvelle richesse et permet d'accéder à une biodiversité chimique « en sommeil ».

Dans l'état de l'art, en général les élicitations (chimiques, physiques, biologiques) de la culture cellulaire permettent de stimuler et de produire plus de métabolites secondaires. Dans notre procédé d'invention, nous démontrons que contrairement à la majorité des cas, et de manière surprenante l'élicitation n'est pas nécessaire mais détrimentale à la croissance de la biomasse et aux activités biologiques recherchées.

Par « cellules végétales dédifférenciées », on entend toute cellule végétale ne présentant aucun caractère d'une spécialisation particulière c'est-à-dire dans un état physiologique proche des tissus méristematiques de la plante à l'état naturel. Ces cellules sont capables de vivre par elles-mêmes et non en dépendance avec d'autres cellules.

Les cellules dédifférenciées d'*Argania spinosa* sont obtenues à partir de matériel végétal vivant prélevé sur l'arbre ou la jeune pousse, que ce soit la feuille, les pétioles, la tige, l'écorce, la racine, le fruit, la graine, la fleur et ses organes ou le bourgeon, plus particulièrement à partir de feuille.

Le procédé d'obtention de la culture de cellules dédifférenciées est obtenue in vitro par toute méthode connue par l'homme de l'art, on se référera par exemple à : Murashige, T., Skoog, F. 1962. A revised médium for rapid growth and bio assays with tobacco tissue cultures. Physiol. Plant 15: 473-496. / Plant Culture Media, Vol-1 Formulations and Uses E. F. George, D. J. M. Puttock, and H. J. George (1987) Exegetics Ltd. Edington, Westbury, Wilts, BA134QG England.

La préparation selon la présente invention peut être obtenue en réalisant les étapes successives suivantes :

a) stérilisation du matériel végétal,
b) mise en dédifférenciation des cellules,
c) mise en suspension cellulaire avec un milieu de culture sans éliciteur,
d) culture de propagation et production de biomasse avec un milieu de culture sans éliciteur,

et obtention de la préparation.

La préparation peut s'effectuer en erlenmeyer s'il s'agit de produire de faibles quantités de biomasse ou en bioréacteur pour des quantités plus importantes. Par exemple, en erlenmeyer avec 500ml de suspension cellulaire, on récolte 100g de biomasse essorée en moyenne (soit 200 g de biomasse /L de suspension cellulaire) alors qu'en bioréacteur de 10L on récolte 3000g de biomasse essorée en moyenne (300g/L de biomasse).

Trois modes principaux sont rencontrés pour la culture de cellules végétales en bioréacteur :

1. la culture discontinue ou batch,
2. la culture recharge/récolte ou fed-batch et
3. la culture continue.

a. Etape de stérilisation du matériel végétal :

Des explants d'*Argania spinosa* et plus particulièrement des explants de feuilles sont prélevées et décontaminées avec des solutions d'hypochlorite de sodium ou de calcium ou bien des solutions de chlorure de mercure à température ambiante durant plusieurs minutes. Les tissus sont rincés à l'eau distillée stérile puis subissent au moins un lavage à l'eau distillée stérile en fin de décontamination.

b. Etape de dédifférentiation des cellules

Les explants décontaminés sont placés sous hotte à flux laminaire en contact avec un milieu gélosé nutritif de Murashige & Skoog, complémenté de saccharose et de facteurs (ou hormones) de croissance. Ces derniers vont conditionner la machinerie cellulaire des explants de manière à provoquer des divisions cellulaires et aboutir à des amas cellulaires ou cals dédifférenciés (callogénèse). Les cals obtenus vont être transférés sur un milieu nutritif de dédifférentiation neuf toutes les 3 à 4 semaines. En effet certain constituants de ce milieu gélosés peuvent se trouver métabolisés par les cals ou bien dégradés par l'action de l'air.

De façon générale, afin d'obtenir une dédifférenciation rapide et poussée des tissus sous la forme de cals friables (callogénèse) pour favoriser un passage en milieu liquide, une composition hormonale à base d'auxine (2-4 dichlorophénoxyacétic acid) et de cytokinine (kinétine) a été testée avec succès. Les explants foliaires stérilisés peuvent être déposés sur la face adaxiale en contact avec le milieu gélosé composé d'un milieu de Murashige et Skoog (Murashige, T., Skoog, F. 1962. A revised medium for rapid growth and bio assays with tobacco tissue cultures. Physiol. Plant 15: 473-496) avec 30g/L de saccharose, 8g/L d'agar, complémenté avec 0,5 mg/L de kinetine et 0.75 mg/L d'acide 2-4 dichloro-phenoxyacetique (24D) et ajusté à pH 6 avant un autoclavage de 20 min à 121°C (1 bar). Les boîtes de pétri contenant les explants sont laissés à incuber à l'obscurité à 28°C. Les premiers cals apparaissent au bout de 2 semaines. Les cals obtenus sont transférés sur un nouveau milieu toutes les 3 - 4 semaines en divisant les cals au scalpel de manière à maintenir une taille de 2 à 3 cm. Ces transferts se succèdent pendant 2 à 6 mois de manière à obtenir des cals friables.

c. Etape de mise en suspension cellulaire dans un milieu de culture sans éliciteur

La dédifférentiation cellulaire au gré des transferts successifs des cals sur milieu gélosé abouti à la formation de cals friables. Cette baisse de la cohésion entre les cellules est une conséquence de la dédifférentiation qui peut survenir entre deux et six mois selon la plante. Cet état est propice au passage en milieu liquide car il garanti un délitement des cals en suspension cellulaire tout en minimisant les stress mécaniques induits. Ainsi une collection de cals friables est introduit (10-20% du volume) dans le milieu nutritif liquide préparé suivant la même formulation que le milieu gélosé de dédifférentiation mais sans agent gélifiant.

Les cals friables sont délités ainsi en milieu liquide par l'action d'une table d'agitation pendant 2 à 3 jours et la suspension cellulaire obtenue est débarrassée des parties de cals non délitées formant ainsi une suspension cellulaire homogène. Cette suspension est maintenue en culture de manière à obtenir une population cellulaire suffisamment dense. A ce stade la suspension est (repiquée ou) diluée dans du milieu nutritif neuf et mise en culture de la même manière.

La mise en suspension cellulaire initiale peut être réalisée en déposant environ 20 à 40 g de cals friables dans un erlenmeyer de 500ml contenant 200 ml de milieu. Les cals friables sont délités ainsi en milieu liquide par l'action d'une table d'agitation pendant 2 à 3 jours à 115 RPM à l'obscurité à 29°C. Ensuite le surnageant cellulaire est récolté à la pipette laissant de coté les amas de cals résiduels non délités. Le surnageant cellulaire forme ainsi une suspension cellulaire homogène. Cette suspension est maintenue en culture de manière à obtenir une population cellulaire « suffisamment » dense. La suspension cellulaire obtenue est cultivée pendant 15 J puis propagée par dilution au 1/5ème dans du nouveau milieu sur la même durée. Des ajustements de la composition du milieu de culture (nutriments, facteurs de croissance etc.) ont été réalisés afin de maximiser la productivité en biomasse. Il en résulte le milieu de propagation de la biomasse ARGMS (Cf tableau 1) optimisé pour la suspension cellulaire liquide. Ce milieu est une version modifiée du milieu de Murashige & Skoog pour la callogénèse. Ce milieu est ajusté à pH 6 par l'ajout de KOH suivi d'un autoclavage de 20 min à 121°C (p = 1 bar) ou d'une filtration stérilisante à 0,2$\mu$m.

Tableau 1 : Milieu ARGMS qui est une version modifiée du milieu de Murashige & Skoog (ARGMS) servant pour la culture en conditions optimales des cellules d'arganier en suspension en erlenmeyer ou en bioréacteur

| Milieu ARGMS - optimisé pour la croissance cellulaire | | | |
|---|---|---|---|
| $NH_4NO_3$ | 2 | g/L | Macro éléments |
| $KNO_3$ | 2 | g/L | |
| $CaCl_2.2H_2O$ | 0,33 | g/L | |
| $MgSO_4.7H_2O$ | 0,25 | g/L | |
| $KH_2PO_4$ | 0,3 | g/L | |
| KI | 0,83 | mg/L | Micro éléments |
| $H_3BO_3$ | 6,2 | mg/L | |
| $MnSO_4.4H_2O$ | 22,3 | mg/L | |
| $ZnSO_4.1H_2O$ | 6,61 | mg/L | |
| $Na_2MoO_4.2H_2O$ | 0,25 | mg/L | |
| $CuSO_4.5H_2O$ | 0,025 | mg/L | |
| $CoCl_2.6H_2O$ | 0,025 | mg/L | |
| $FeSO_4.7H_2O$ | 41,7 | mg/L | |
| $Na2EDTA.2H_2O$ | 55, 95 | mg/L | |
| myo-Inositol | 150 | mg/L | Vitamines |
| Acide nicotinique | 0,75 | mg/L | |
| Pyridoxine-HCl | 0,75 | mg/L | |
| Thiamine-HCl | 0,75 | mg/L | |
| Glycine | 2 | mg/L | |
| 24D | 0,75 | mg/L | Facteurs (hormones de croissance) |
| Kinétine | 0,5 | mg/L | |
| Saccharose | 35 | g/L | Sources carbonées |
| Pyruvate de Na | 3 | g/L | |

d. culture de propagation et production de biomasse avec un milieu de culture sans éliciteur

Après plusieurs repiquages de la sorte la suspension cellulaire est stabilisée lorsque la densité de cellules obtenue sur la période est constante. Des ajustements de la composition du milieu de culture (nutriments, facteurs de croissance...) est alors possible afin de maximiser la productivité en biomasse. Dans un mode de réalisation particulier de l'invention, le milieu optimisé employé comme moyen de production de biomasse est le milieu décrit dans le tableau 1.

La suspension cellulaire est filtrée afin de la séparer du milieu extracellulaire ou surnageant de culture et la biomasse récoltée est remise en suspension dans de l'eau distillée et broyée à 0°C. Le broyat obtenu est lyophilisé ou bien centrifugé de manière le clarifier avant d'être lyophilisé

La culture cellulaire en condition « optimale » ainsi établie est stabilisée et entretenue en erlenmeyer (culture de propagation) à raison d'une dilution de la suspension cellulaire au 1/5ème tous les 15 jours. Ceci équivaut à une culture cellulaire inoculée à 60g/L env. de biomasse fraîche qui produit une suspension cellulaire de 300g/L env. au bout de 15 jours de culture, ou inoculée en bioréacteur selon les besoins.

Que ce soit en erlenmeyer ou en bioréacteur, la préparation obtenue peut consister en :

➢ une suspension cellulaire (par « suspension cellulaire » on entend au sens de la présente invention, les cellules ou biomasse dans leur milieu de culture)
➢ de la biomasse (par « biomasse » on entend au sens de la présente invention un amas cellulaire séparé du milieu de culture ; soit la suspension cellulaire après filtration)
➢ de la biomasse broyée après remise (ou non) en suspension dans de l'eau distillée

➢ un jus clarifié ou surnageant de biomasse broyée par centrifugation ou par filtration

➢ un surnageant de culture (par « surnageant de culture » on entend au sens de la présente invention, le milieu de culture dans lequel les cellules ont séjournés lors de la culture ou milieu extracellulaire).

Que ce soit la suspension cellulaire, la biomasse ou le surnageant de biomasse broyée, ils peuvent être conservés tels quels sous forme congelée ou par l'addition de substances conservatrices telles que le phénoxy-2-éthanol, l'alcool benzylique ou tout autre conservateur figurant dans l'annexe VI de la directive de l'UE relative aux produits cosmétiques intitulée « Liste des agents conservateurs que peuvent contenir les produits cosmétiques ». Ils peuvent également être dilués sur un support cosmétologiquement acceptable du type glycol (propylène glycol, butylène glycol, polyéthylène glycols..) dans des proportions allant de 10 à 60%. La suspension cellulaire ou la biomasse peuvent également être broyées puis conservées telles quelles congelées, ou par l'addition de substances conservatrices ou de support comme décrit ci-dessus.

Broyés ou non, la suspension cellulaire, la biomasse ou le surnageant de biomasse peuvent également être séchés par lyophilisation ou atomisation, et conservés tels quels ou séchés sur un support de type maltodextrine, lactose, silice, ou tout autre support cosmétologiquement acceptable.

Enfin, la suspension cellulaire peut être enrichie en composés d'intérêt par chromatographie d'affinité : absorption sur résine (copolymères de polystyrène type Amberlite® XAD®-2, etc.) et élution avec un solvant approprié tel que l'éthanol.

La biomasse fraîche, obtenue avec le procédé selon la présente invention, représente environ 100 à 500g par litre de suspension, et de façon plus préférentielle entre 200 et 350 g par litre de suspension à la date de récolte optimale (soit environ 15 jours en moyenne).

Le tableau suivant exprime les rendements obtenus (rendement en grammes de produit obtenu / L de suspension cellulaire) :

| Produit | Biomasse fraîche | | Biomasse fraîche broyée lyophilisée | | Surnageant de biomasse fraîche broyée lyophilisée | |
|---|---|---|---|---|---|---|
| Modes de culture | Productions (préféré) | Productivités (préféré) | Productions (préféré) | Productivités (préféré) | Productions (préféré) | Productivités (préféré) |
| Erlenmeyer | 150 - 300 $g.L^{-1}$ en 15 jours (200 $g.L^{-1}$ en 15 jours) | 10-20 $g.L^{-1}.j^{-1}$ (13 $g.L^{-1}.j^{-1}$) | 7,1 - 15 $g.L^{-1}$ en 15 jours (9,5 $g.L^{-1}$ en 15 jours) | 0,4-1 $g.L^{-1}.j^{-1}$ (0,6 $g.L^{-1}.j^{-1}$) | 4,5 - 9 $g.L^{-1}$ en 15 jours (6 $g.L^{-1}$ en 15 jours) | 0,3-0,6 $g.L^{-1}.j^{-1}$ (0,4 $g.L^{-1}.j^{-1}$) |
| Bioréacteur 10L-Batch | 200 - 500 $g.L^{-1}$ en 15 jours (300 $g.L^{-1}$ en 15 jours) | 13-34 $g.L^{-1}.j^{-1}$ (20 $g.L^{-1}.j^{-1}$) | 9,5 - 24 $g.L^{-1}$ en 15 jours (14,3 $g.L^{-1}$ en 15 jours) | 0,65-1, 14 $g.L^{-1}.j^{-1}$ (0,95 $g.L^{-1}.j^{-1}$) | 6 - 15 $g.L^{-1}$ en 15 jours (9 $g.L^{-1}$ en 15 jours) | 0,4-1 $g.L^{-1}.j^{-1}$ (0,6 $g.L^{-1}.j^{-1}$) |
| Bioréacteur 10L-Fed Batch de 80% | 200 - 500 $g.L^{-1}$ (300 $g.L^{-1}$ à chaque récolte de 6 jours) | 32-80 $g.L^{-1}.j^{-1}$ (48 $g.L^{-1}.j^{-1}$) | 9,5 - 24 $g.L^{-1}$ (14,3 $g.L^{-1}$ à chaque récolte de 6 jours) | 1,5-3,85 $g.L^{-1}.j^{-1}$ (2,28 $g.L^{-1}.j^{-1}$) | 6 - 15 $g.L^{-1}$ (9 $g.L^{-1}$ à chaque récolte de 6 jours) | 0, 96-2,4 $g.L^{-1}.j^{-1}$ (1,44 $g.L^{-1}.j^{-1}$) |
| Bioréacteur 10L-culture continue $\mu=0,2\ j^{-1}$ | 100-500 $g.L^{-1}$ (140 $g.L^{-1}$ en continu) | 20-100 $g.L^{-1}.j^{-1}$ (28 $g.L^{-1}.j^{-1}$) | 4,8 - 24 $g.L^{-1}$ (6,6 $g.L^{-1}$ en continu) | 1-5 $g.L^{-1}.j^{-1}$ (1,33 $g.L^{-1}.j^{-1}$) | 3 - 15 $g.L^{-1}$ (4,2 $g.L^{-1}$ en continu) | 0,6-3 $g.L^{-1}.j^{-1}$ (0,84 $g.L^{-1}.j^{-1}$) |

La présente invention concerne également l'utilisation d'une composition cosmétique ou dermatologique comprenant à titre de principe actif une préparation issue d'une culture de cellules dédifférenciées non élicitées d'Arganier telle que décrite précédemment dans le traitement non thérapeutique du vieillissement cutané. De préférence, la quantité de ladite préparation est comprise entre 0,1 et 10 % par rapport au poids total de la composition. Et plus préférentiellement, ladite quantité d'extrait est comprise entre 0,2 % et 5 %.

La composition cosmétique selon la présente invention peut avantageusement se présenter sous toutes les formes galéniques normalement utilisées dans le domaine cosmétique pour une application topique ou orale, et préférentiellement topique. Pour une administration par voie topique, la forme galénique peut être une crème, un gel, une pommade, un spray. La forme orale est choisie parmi le groupe comprenant des comprimés, des gélules et des poudres pour suspensions buvables.

La composition cosmétique selon l'invention comprend, en outre, des excipients usuels cosmétiquement compatibles.

Les excipients usuels compatibles à la composition cosmétique peuvent être tout excipient parmi ceux connus de l'homme du métier en vue d'obtenir une composition cosmétique pour une application topique sous les formes telles que décrites ci-dessus.

La composition cosmétique et/ou dermatologique selon l'invention peut en particulier contenir des additifs et aides à la formulation, tels que des tensio-actifs de type émulsionnant, nettoyant, moussant, etc., des agents complexants, des épaississants, des gélifiants, des stabilisants, des conservateurs dont des antimicrobiens et des antioxydants, des conditionneurs, des acidifiants, des alcalinisants, des émollients, des solvants, des colorants, des parfums.

Les inventeurs ont montré que des préparations issues de culture de cellules dédifférenciées non élicitées d'Arganier possèdent les activités suivantes :

> ➢ une activité anti-oxydante, anti-radicalaire pour limiter le processus d'oxydation lié au vieillissement intrinsèque et extrinsèque et le processus inflammatoire.
> ➢ une activité sur la matrice extra-cellulaire pour améliorer les propriétés mécaniques de la peau mature (fermeté, élasticité, tonicité) via l'inhibition de metalloprotéases dégradant le collagène.

Les exemples suivants sont donnés à titre indicatif non limitatif.

Exemples d'obtention de la préparation selon la présente invention

Exemple 1 : biomasse fraîche / procédé mené en erlenmeyer

Des feuilles d'Arganier âgées de 3 à 4 mois de préférence sont stérilisées par les bains successifs suivants : alcool à 70% 1 min, hypochlorite de sodium à 2% 3 min puis rincés par deux bains successifs d'eau déminéralisée de 8 min et 10 min.

Les explants foliaires stérilisés sont déposés sur la face adaxiale en contact avec le milieu gélosé composé d'un milieu de Murashige et Skoog (Murashige, T., Skoog, F. 1962. A revised medium for rapid growth and bio assays with tobacco tissue cultures. Physiol. Plant 15: 473-496) avec 30g/L de saccharose, 8g/L d'agar, complémenté avec 0,5 mg/L de kinetine et 0.75 mg/L d'acide 2-4 dichloro-phenoxyacetique (2,4-D) et ajusté à pH 6 avant un autoclavage de 20 min à 121°C (1 bar). Les boîtes de pétri contenant les explants sont laissés à incuber à l'obscurité à 28°C, et propagés jusqu'à l'obtention de cals friables et stabilisés.

La mise en suspension cellulaire initiale est réalisée en déposant environ 40 g de cals friables dans un erlenmeyer de 500ml contenant 200 ml de milieu autoclavé dont la composition est détaillée dans le tableau 1 précité.

La culture est réalisée pendant une semaine sur une table d'agitation à 115 RPM à l'obscurité à 29°C. Ensuite le surnageant cellulaire est récolté à la pipette laissant les amas de cals résiduels. La suspension cellulaire obtenue est cultivée pendant 15 J puis propagée par dilution au 1/5ème dans du nouveau milieu sur la même durée.

La suspension est ensuite filtrée sous vide et la biomasse récupérée. Le rendement de biomasse fraîche obtenu est de 168g/L.

La biomasse est conservée à -20°C.

Exemple 2 : biomasse sèche

Exemple 2a : biomasse sèche / procédé mené en bioréacteur en culture batch

Quatre erlenmeyers de suspension cellulaire de 500ml obtenus tel que décrit dans l'exemple 1 sont regroupés dans un dispositif inoculateur et forment un inoculum de 2L qui est déversé stérilement dans un bioréacteur de 10L. Ce bioréacteur est rempli de 8L de milieu optimal (voir tableau 1) complémenté de 30mg/L d'antimousse préalablement stérilisé puis refroidi et maintenu à 29,5°C par une circulation d'eau thermostatée en circuit fermé dans l'enveloppe du bioréacteur.

Une sonde à oxygène est étalonnée par saturation et renseigne en temps réel un dispositif informatisé de régulation de la pO2. Celui-ci permet de maintenir la pO2 à 80% par injection d'oxygène pur stérile dans le dispositif d'aération. Ce bioréacteur est équipé également d'un dispositif de mesure en ligne du CO2 au niveau des gaz effluents (head space) qui renseigne en temps réel un dispositif informatisé de régulation de la pCO2 de manière à maintenir la pCO2 à 6%. Cette dernière est faite par injection d'air atmosphérique stérile dans le dispositif d'aération en mélange avec l'oxygène. Le bioréacteur est également équipé d'un système d'agitation à pale marine tournant à 75RPM de manière suffisante pour brasser la suspension cellulaire et éviter qu'elle ne sédimente. En aval du bioréacteur est installé un dispositif automatique permettant un prélèvement stérile de la biomasse et un suivi de celle-ci.

La culture batch est maintenue dans ces conditions de température et de gaz dissous constantes pendant 15 à 17 jours de manière à atteindre une densité cellulaire de 280 à 320g/L de biomasse fraîche. A l'issue de cette culture en batch, le bioréacteur est vidé et la biomasse récoltée par filtration sur un filtre à l'aide d'un entonnoir Büchner.

La biomasse fraîche récoltée reprise dans le même volume d'eau distillée est broyée à froid à l'aide d'un sonicateur puis lyophilisée.

Exemple 2b : biomasse sèche / procédé mené en bioréacteur en culture Fed-batch

Quatre erlenmeyers de suspension cellulaire de 500ml sont utilisés comme inoculum tel que décrit dans l'exemple 2a. Le bioréacteur est préparé comme indiqué dans l'exemple 2a. Les dispositifs de régulation des gaz dissous, de la température et de l'agitation sont préparés comme indiqué dans l'exemple 2a.

La culture initiale est maintenue dans ces conditions de température et de gaz dissous constantes pendant 15 à 17 jours de manière à atteindre une densité cellulaire de 280 à 320g/L de biomasse fraîche. A l'issue de cette culture en initiale, le bioréacteur de 10L est vidé à 80%. On récolte alors 8L suspension cellulaire. La biomasse de cette suspension est récoltée par filtration sur un filtre à l'aide d'un entonnoir Büchner. On récolte 2240g à 2560g de biomasse fraîche. La biomasse fraîche récoltée reprise dans le même volume d'eau distillée est broyée à froid à l'aide d'un sonicateur puis lyophilisée. On obtient 100 à 130g de biomasse lyophilisée. Simultanément à la récolte partielle de 80% on déverse 8L de milieu ARGMS préalablement autoclavé et refroidi dans le bioréacteur contenant alors 2L de suspension cellulaire de manière à rétablir le volume de culture de 10L. La culture Fed-batch est maintenue dans ces conditions de température et de gaz dissous constantes pendant 5 à 7 jours de manière à atteindre une densité cellulaire de 280 à 320g/L de biomasse fraîche. Cette culture est plus courte (plus grande productivité) que la culture initiale car la biomasse est dans un état physiologique de division cellulaire soutenu si bien que le deversement de milieu nutritif neuf se caractérise par une phase de latence de moins de 24 heures et une expansion immédiate de la biomasse. A l'issue de cette culture en Fed-batch, le bioréacteur de 10L est vidé à 80%. On récolte alors 8L suspension cellulaire. La biomasse de cette suspension est récoltée par filtration sur un filtre à l'aide d'un entonnoir Büchner. La culture est ensuite relancée comme précédemment.

Exemple 2c : biomasse sèche / procédé mené en bioréacteur en culture Continue

Quatre erlenmeyers de suspension cellulaire de 500ml sont utilisés comme inoculum tel que décrit dans l'exemple 2a. Le bioréacteur est préparé comme indiqué dans l'exemple 2a. Les dispositifs de régulation des gaz dissous, de la température et de l'agitation sont préparés comme indiqué dans l'exemple 2a.

La culture initiale est maintenue dans ces conditions de température et de gaz dissous constantes pendant 10 jours de manière à atteindre une densité cellulaire de 150g/L et un taux de croissance instantané de la biomasse fraîche de 0,2 $j^{-1}$. A ce stade le bioréacteur de 10L est vidé à 1,2 % toutes les 1 heures et 20 minutes. Ces prélèvements sont compensés automatiquement par le déversement de même volume de milieu ARGMS neuf dans le bioréacteur. Cette méthode permet de maintenir les cellules dans un état physiologique et une densité cellulaire constante.

On récolte alors 100 à 120ml de suspension cellulaire. La biomasse de cette suspension est récoltée par filtration sur un filtre à l'aide d'un entonnoir Büchner. On récolte 15g à 18g de biomasse fraîche à chaque prélèvement. La biomasse fraîche récoltée reprise dans le même volume d'eau distillée est broyée à froid à l'aide d'un sonicateur puis lyophilisée. On obtient 0,71 à 0,85g de biomasse lyophilisée à chaque prélèvement. La culture est ainsi maintenue, pendant au moins 60 jours. Il est théoriquement possible de la maintenir sans limite de durée. L'avantage de la culture en mode continu rapport aux modes précédents est l'absence de re-préparation du bioréacteur consistant en un nettoyage et une stérilisation de celui-ci ainsi que l'absence de phase de latence cellulaire. En effet les prélèvements de 1 à 1.5% de la suspension cellulaire suivi automatiquement par la compensation en milieu neuf dans le bioréacteur inflige des variations minimes de composition du milieu de culture en cours dans le bioréacteur. Ainsi la population cellulaire ne procède pas aux réajustements métaboliques de la phase de latence responsable d'une perte de productivité volumique en biomasse observé dans les autres modes de culture.

Exemple 3 : surnageant de biomasse fraîche, ladite biomasse étant obtenue selon l'exemple 2a

20 g de biomasse fraîche et broyée obtenue selon l'exemple 2a sont centrifugés à 10000g pendant 15 minutes, et le surnageant est récolté. Il est ensuite lyophilisé.

Le rendement moyen est de 30mg de surnageant lyophilisé par g de biomasse fraîche.

Exemples de compositions cosmétiques :

Exemple 4 : Formule H/E

| Composants | % |
|---|---|
| Biomasse fraîche (exemple 1) | 5 |
| Glycérine | 10.0 |
| Na2EDTA | 0.1 |
| Gomme xanthane | 0.3 |
| C12-C15 alkyl benzoate | 10.0 |
| Octyl palmitate | 5.0 |
| Conservateurs | qs |
| Alcool stéarique | 2.5 |
| Monostéarate de glycérol | 2.5 |
| Potassium cetyl phosphate | 1.8 |
| Eau déminéralisée | QSP 100 |

Exemple 5 : Formule E/H

| Composants | % |
|---|---|
| Surnageant (exemple 3) | 0,5 |
| Glycérine | 4,0 |
| Na2EDTA | 0,1 |
| $MgSO_4$ | 1,0 |
| Gomme xanthane | 0,1 |
| C12-C15 alkyl benzoate | 12,5 |
| Isohexadécane | 3,5 |
| Cyclométhicone | 3,0 |
| Conservateurs | qs |
| Esters de polyglycérol et de sorbitan | 4,0 |
| Myreth-3 myristate | 2,0 |
| Eau déminéralisée | QSP 100 |

Exemple 6 : EVALUATION DE L'ACTIVITE ANTI-OXYDANTE Chemiluminescence

C'est une méthode qui génère des radicaux libres (radical superoxide $O_2^{\circ-}$) par un signal photochimique.

L'intensité de l'oxydation est 1000 fois supérieure à celle obtenue en conditions normales. La détection se fait par chemiluminescence et permet l'évaluation d'extraits ou de molécules anti-oxydants lipo- ou hydrosolubles. Les résultats sont exprimés respectivement en quantité équivalente de vitamine C ou de Trolox (6-Hydroxy-2,5,7,8-tetramethylchroman-2-carboxylic Acid). La sensibilité est de l'ordre de la nanomole.

L'analyse des résultats dépend de 2 critères : de l'allure de la courbe (intégration) et de la valeur numérique en nanomole donnée par le logiciel. (Igor Popov and Gudrun Lewin. Methods in enzymology [44] vol 300. 437-456 ; Maibach I Howard and coll. Journal of Cosmetic Dermatology Vol 7(2) 96-100 (2008)).

Les résultats seront exprimés en µg d'échantillon nécessaire pour obtenir une activité équivalente à l'activité détectée pour 1µg de standard (= trolox). (Kit ACL).

Résultats :

L'activité anti-oxydante étudiée dans ce test représente la capacité à piéger spécifiquement les anions superoxydes par chemiluminescence.

Tableau 2: Evaluation et quantification du pouvoir anti - oxydant en équivalent trolox.

| Echantillon testé | ACL |
|---|---|
| | µg d'échantillon pour 1µg de Trolox |
| Trolox (référent) | 1 |
| Surnageant (exemple 3) | 171 |
| Coenzyme 10 = Molécule antioxydante de référence | 272 |
| Biomasse (exemple 2a) | 278 |

La biomasse lyophilisée préparée selon l'exemple 2a et le surnageant de biomasse broyée lyophilisé préparé selon l'exemple 3 ont une activité vis à vis du piégeage anti radicalaire globalement équivalente.

278 µg de biomasse lyophilisée est nécessaire pour obtenir une activité équivalente à l'activité détectée pour 1 µg de trolox : activité équivalente au coenzyme Q10 molécule anti-oxydante de référence.

171 µg de surnageant de biomasse broyée lyophilisé est nécessaire pour obtenir une activité équivalente à l'activité détectée pour 1 µg de trolox.

Les radicaux libres, dont la production est accrue suite aux agressions extérieures (froid, pollution, tabac, UV) sont responsables des altérations de l'ADN des cellules cutanées, mais aussi des membranes cellulaires et mitochondriales. Ces radicaux libres jouent un rôle très important également dans le processus d'inflammation. Ces métabolites très réactifs sont des seconds messagers de la signalisation cellulaire du stress oxydatif et donc médiateurs précoces de l'inflammation (A. Van Der Vliet and coll, Chem Biol Interaction 85 : 95- 116 1992). L'activité anti-radicalaire des préparations décrites dans les exemples 2a et 3 permettent de lutter contre le vieillissement cutané intrinsèque et extrinsèque et contre l'inflammation.

Exemple 7 : EVALUATION DE L'INHIBITION DE L'ACTIVITE METALLOPROTEASIQUE SUR LE COLLAGENE CONSTITUANT DE LA MATRICE EXTRA-CELLULAIRE.

La matrice extracellulaire (MEC) est une structure dynamique ayant un rôle structurel et régulateur pour les tissus. Elle confère à la peau sa turgescence et ses propriétés mécaniques. Au niveau de l'épiderme elle occupe l'espace intercellulaire et joue un rôle de maintien pour l'édifice épidermique. Elle assure également les échanges entre les cellules de l'épiderme et joue un rôle dans l'activité cellulaire. Elle est constituée de fibres, notamment de collagène et de substance fondamentale (eau, sels, glycoprotéines, glycosaminoglycanes). Les collagènes sont des protéines fibreuses, formées de trois chaînes polypeptidiques, identiques ou différentes, reliées par des liaisons covalentes et hydrogènes. Les collagènes constituent le composant essentiel du réseau fibreux et jouent un rôle mécanique conférant résistance et élasticité de la peau.

Lorsqu'une cellule est sénescente, les composants de la MEC sont majoritairement dégradés par des enzymes du type endopeptidases à zinc appelés métalloprotéinases matricielles ou MMPs (Hideaki Nagase § and J. Frederick Woessner. J Biol Chem, Vol. 274, Issue 31, 21491-21494, July 30, 1999). Elles sont membranaires ou sécrétées. Toutes les MMPs ont une forte homologie de séquence et de structure mais se distinguent par la spécificité de substrat. La MMP1 ou « collagénase interstitielle » dégrade majoritairement le collagène de type I (présent à 80% dans le derme d'une peau normale) et dégrade également les collagènes II VII VIII et X.

Sur un modèle d'enzyme recombinante humaine à l'aide d'un substrat peptidique spécifique Mca - Lys-Pro-Leu-Gly-Leu-DPA-Ala-Arg-NH2, nous avons analysé l'effet des extraits sur l'activité enzymatique directe par un dosage fluorimétrique. (David Leppertd and coll , Analytical Biochemistry 328 (2004) 166-17).

L'enzyme activée est pré incubée avec les différentes préparations puis mise en présence du substrat. L'enzyme clive le peptide séparant le fluorophore Mca (7 methoxycoumarin -4-yl) acethyl) du quencher Dpa (N - 3 - (2,4-Dinitrophenyl) - L-2,3 diaminopropionyl). Le peptide émet alors une fluorescence d'une longueur d'onde de 405nm lorsqu'il est excité à 320nm. Ainsi, l'activité enzymatique de la MMP-1 est mesurée et est proportionnelle à la fluorescence émise.

Par ce présent test in tubo, nous pouvons détecter de potentiels inhibiteurs d'activité MMP1, enzyme ayant un rôle crucial dans l'initiation de la dégradation des collagènes. Nous mesurons des pourcentages d'inhibition d'activité MMP1.

Calcul du pourcentage d'inhibition enzymatique liée à l'inhibiteur ou au produit :

$$\% \text{ inhibition} = 100 \text{ x } \frac{(Activité\ nette\ enzymatique\ \max imale - Activité\ nette\ enzymatique\ en\ présence\ d'inhibiteur)}{Activité\ nette\ enzymatique\ \max imale}$$

Résultats :

Le surnageant préparé selon l'exemple 3 inhibe l'activité MMP1 de façon significative et dose dépendante de 60 à 500 µg/ml.

Tableau 3: Surnageant de biomasse broyée lyophilisé (préparé selon l'exemple 3), résultats de l'inhibition de la MMP1 en pourcentage (%).

| Moyenne % d'inhibition | Concentration en µg/ml |
|---|---|
| 100 | 500 |
| 58 | 300 |
| 41 | 150 |
| 20 | 60 |

La biomasse lyophilisée préparée selon l'exemple 2a a été testée de 60 à 1000µg/ml. En raison d'interférences physico chimiques de la biomasse dans son ensemble, nous n'avons pas pu mesurer l'activité inhibitrice. Toutefois, un extrait très proche ayant été testé a montré des inhibitions significatives de 60µg/ml à 1000µg/ml.

L'extrait préparé selon l'exemple 3 permet de lutter contre l'activité accrue des MMPs lors de la sénescence et de contribuer à la conservation du rôle mécanique du collagène, conférant résistance et élasticité au niveau de la peau.

Exemple 8 : Mesure de la synthèse de TGF- β1 chez les kératinocytes HaCaT

Le TGF-β1 (Transforming Growth Factor-beta 1) appartient à la super-famille des TGF-β .qui sont sécrétés par différents types cellulaires et qui jouent un rôle important dans le contrôle de la croissance cellulaire et la régulation de multiples réponses cellulaires et processus biologiques. Les cytokines de cette super-famille possèdent les activités majeures suivantes : elles modulent la prolifération de la plupart des cellules, elles stimulent la prolifération des fibroblastes, elles augmentent la formation de la matrice extracellulaire (Lawrence, 1996). Par ailleurs, le TGF-β1 est impliqué dans la réparation des blessures, les processus de cicatrisation (Cullen et al., 1997) notamment en induisant une réorganisation du cytosquelette cellulaire (actine) et en promouvant la migration des cellules épithéliales (Boland et al., 1996). La population cellulaire la plus représentée au niveau du tissu cutané est la population kératinocytaire. Elle constitue une source importante de facteurs de croissance susceptibles de réguler et influencer le comportement des cellules dermiques : les fibroblastes (Ghahary et al., 2001).

Matériels et Méthodes

Production de biomasse non élicitée : selon l'exemple 2a Préparation de biomasse élicitée

Du milieu de Murashige & Skoog est préparé sans facteurs de croissance (kinétine et 24D) Ce milieu est ajusté à pH 6 par l'ajout de KOH suivi d'un autoclavage de 20 min à 121°C (p = 1 bar). Ce milieu est ensuite inoculé au 1/5ème du volume à l'aide de suspension cellulaire issue d'une culture de propagation. Les conditions d'élicitation sont réalisées immédiatement après par l'ajout stérile de solution concentrée dans du DMSO de kinétine, de 6-benzylaminopurine (BAP ou (N-(phénylméthyl)-7H-purin-6-amine) et d'agents éliciteurs : acide acétylsalicylique et méthyl-jasmonate. Il en résulte un milieu EMS d'élicitation (Cf Tableau 4). La culture élicitée est ensuite maintenue pendant 15 jours sur une table d'agitation à 115 RPM à l'obscurité à 29°C. La biomasse fraîche est ensuite récoltée et essoré sur entonnoir buchner avant d'être broyée, centrifugée et le surnageant stabilisé par lyophilisation.

Tableau 4. Milieu EMS qui est le milieu de Murashige & Skoog modifié servant pour la culture en condition élicitée des cellules d'arganier en suspension en erlenmeyer.

| Milieu EMS - milieu d'élicitation | | | |
|---|---|---|---|
| $NH_4NO_3$ | 1,650 | g/L | Macro éléments |
| $KNO_3$ | 1,900 | g/L | |
| $CaCl_2.2H_2O$ | 0,44 | g/L | |
| $MgSO_4.7H_2O$ | 0,37 | g/L | |
| $KH_2PO_4$ | 0,17 | g/L | |
| KI | 0,83 | mg/L | Micro éléments |
| $H_3BO_3$ | 6,2 | mg/L | |
| $MnSO_4.4H_2O$ | 22,3 | mg/L | |
| $ZnSO_4.1H_2O$ | 6,62 | mg/L | |
| $Na_2MoO_4.2H_2O$ | 0,25 | mg/L | |
| $CuSO_4.5H_2O$ | 0,025 | mg/L | |
| $CoCl_2.6H_2O$ | 0,025 | mg/L | |
| $FeSO_4.7H_2O$ | 27,8 | mg/L | |
| $Na2EDTA.2H_2O$ | 37,8 | mg/L | |
| myo-Inositol | 100 | mg/L | Vitamines |
| Acide nicotinique | 0,5 | mg/L | |
| Pyridoxine-HCl | 0,5 | mg/L | |
| Thiamine-HCl | 0,5 | mg/L | |
| Glycine | 2 | mg/L | |
| 6-benzylaminopurine (BAP) | 1 | mg/L | Facteurs (hormones de croissance) |
| Kinétine | 1 | mg/L | |
| Acide acétylsalicylique | 100 | $\mu$M | Agents éliciteurs |
| Méthyl-jasmonate | 100 | $\mu$M | |
| Saccharose | 35 | g/L | Sources carbonées |
| Pyruvate de Na | 3 | g/L | |

Les kératinocytes HaCaT sont traités 5h avec les différents extraits, puis les cellules sont incubées pendant 24h dans du DMEM à 37°C. Le TGF-$\beta$1 est dosé dans les surnageants de culture avec un kit ELISA.

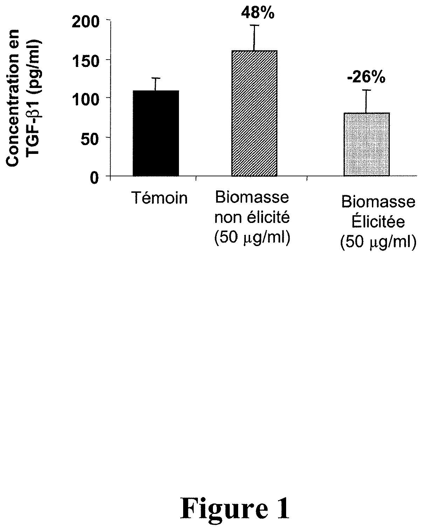

Les effets de la biomasse d'argan non élicitée préparée selon l'exemple 2a et de biomasse d'argan obtenue après élicitation, sur la synthèse de TGF-$\beta$1 chez les kératinocytes humains HaCaT sont illustrés à la figure 1 annexée. Ils montrent que, chez les kératinocytes HaCaT, la biomasse d'argan non élicitée préparée selon l'exemple 2a (50$\mu$g/mL) stimule la synthèse de TGF-$\beta$1 de 48 % alors que la biomasse d'argan élicitée inhibe la synthèse de TGF-$\beta$1 de 26%.

Exemple 9 : Mesure de la prolifération et migration cellulaire des kératinocytes humains

Exemple 9.a : Mesure de la prolifération cellulaire des kératinocytes humains

La cicatrisation des plaies est un processus biologique complexe et dynamique qui met en jeu l'interaction de nombreux facteurs locaux et systémiques dans la réparation normale des tissus. La progression de la cicatrisation comporte quatre phases interdépendantes : l'hémostase, l'inflammation, la prolifération et le remodelage. La prolifération implique trois processus bien observables : la granulation, la contraction et la réépithélialisation.

Au cours de la granulation, on observe la prolifération et la migration vers le lit de la plaie des cellules qui interviendront dans le reste du processus de réparation. Ainsi, on y retrouve des macrophages, des fibroblastes et des cellules endothéliales. Les macrophages libèrent constamment des facteurs chimiotactiques et des facteurs de croissance. Les fibroblastes construisent la nouvelle matrice cellulaire nécessaire à la croissance des cellules au fond de la plaie. Cet échafaudage favorise la migration cellulaire.

La contraction de la plaie est un mécanisme de réduction de la taille de la plaie et les fibroblastes jouent un rôle de premier plan dans cette contraction.

La réépithélisation consiste en la régénération d'un épiderme qui recouvre une plaie pour reformer une barrière efficace contre l'environnement extérieur, capable de se pigmenter et de retrouver ses fonctions sensorielles et immunitaires. Elle implique donc les processus cellulaires de migration et de prolifération des kératinocytes, mais aussi la différenciation de ce néo-épithélium et la restauration d'une membrane basale qui reconnecte le derme et l'épiderme. Lorsque la migration des cellules basales en direction du centre de la plaie permet aux deux bords de la plaie de se rejoindre, une vague de mitose cellulaire se produit pour combler les espaces laissés par la migration et fournir des cellules pour le tissu épithélial en régénération tridimensionnelle.

Les étapes de prolifération des cellules kératinocytaires, de fibroblastes ou de cellules endothéliales peuvent être considérées comme un des phénomènes fonctionnels témoignant de l'activité cicatrisante d'un actif. Une augmentation de la prolifération des fibroblastes ou des cellules endothéliales participerait à la cicatrisation du derme, alors que l'augmentation de la prolifération des kératinocytes participerait à la ré-épithélisation.

Matériels et Méthodes : prolifération cellulaire

La technique utilisée permet de mesurer l'incorporation d'un nucléotide, la 5-bromo-2'-déoxyuridine (BrdU), analogue de la thymidine, dans l'ADN des cellules en phase S.

Les kératinocytes, isolés de déchets opératoires cutanés, sont cultivés dans du KSFM complet (BPE: 25 $\mu$g/ml; EGF: 1,5 mg/ml). Les cellules sont incubées en présence des molécules à évaluer pendant 48h à 37°C, dans une atmosphère à 5% de CO2. L'incorporation du BrdU, proportionnelle au taux de prolifération cellulaire, est évaluée par un système d'anticorps anti-BrdU couplés à la péroxydase. L'addition d'un substrat de la péroxydase développe une réaction colorée (Biotrak Elisa System). L'absorbance correspondante (DO) est mesurée à 450nm. Cette donnée est donc proportionnelle au taux de prolifération cellulaire.

Le pourcentage de prolifération est alors déterminé selon la formule :

$$\% \text{ prolifération} = \frac{\text{DO traité}) - \text{DO (témoin}_{min})}{\text{DO (témoin}_{max}) - \text{DO (témoin}_{min})} \text{ X } 100$$

Remarque :

Témoin$_{min}$ = cellules incubées avec le milieu minimum
Témoin$_{max}$ - cellules incubées avec le milieu complet

Ainsi, le Témoin$_{min}$ correspond au 0% de prolifération, et le Témoin$_{max}$ au 100% de prolifération.

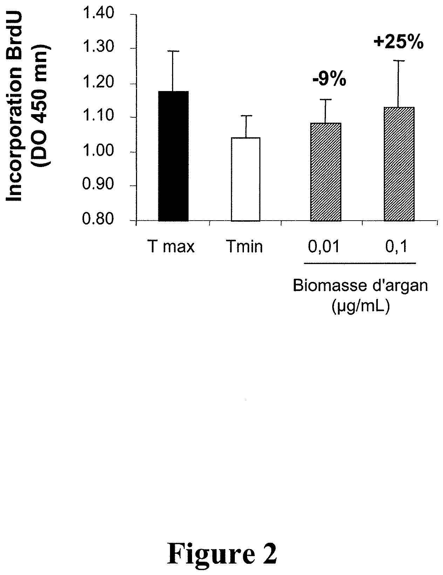

Les résultats sur la prolifération cellulaire sont rassemblés sur la figure 2 qui illustre l'effet de biomasse d'argan préparée selon l'exemple 2a sur la prolifération de kératinocytes humains.

Ils montrent que la biomasse d'argan préparée selon l'exemple 2a, à 0,1 $\mu$g/mL, stimule la prolifération des kératinocytes humains de 25%. Aucun effet n'est mesuré lorsque la biomasse est testée à 0,01 $\mu$g/mL.

Exemple 9.b : Mesure de la migration cellulaire des kératinocytes humains

Matériels et Méthodes : migration cellulaire des kératinocytes HaCaT

Le protocole utilisé pour l'étude de la migration cellulaire repose sur l'utilisation d'un kit 96 puits. Le principe de ce test consiste à étudier la migration des cellules vers le centre du puits (plaque de 96 puits). Pour cela, un stoppeur est placé au centre de chaque puits, afin de créer une zone de détection de 2 mm de diamètre. Les cellules HaCaT sont ensuite ensemencées autour de ce stoppeur. Les stoppeurs sont retirés une fois que les cellules ont bien adhéré à la surface autour de ceux-ci, et ainsi, les cellules peuvent migrer vers la zone de détection. Les plaques sans les stoppeurs et avec les actifs sont mises à incuber à 37°C pendant 24 heures dans du DMEM 0% SVF. On analyse ensuite la quantité de cellules situées dans la zone où il y avait le stoppeur, afin d'évaluer la migration des cellules. Les cellules sont marquées au Hoechst 33342 et un cache permet de visualiser et de comptabiliser uniquement les cellules situées dans cette zone. Pour chaque condition, la moyenne de 8 puits est réalisée.

Les résultats sont exprimés:

➢ en intensité de fluorescence (IF - proportionnelle à la quantité des cellules ayant migré).
➢ en pourcentage d'activité par rapport au témoin 0% SVF :

$$\frac{\text{IF traité - IF 0\%mig}}{\text{IF témoin 0\% SVF - IF 0\% mig}} \times 100$$

Remarque:

IF (0% mig) correspond à l'IF des puits contenant les stoppeurs et donc au bruit de fond.

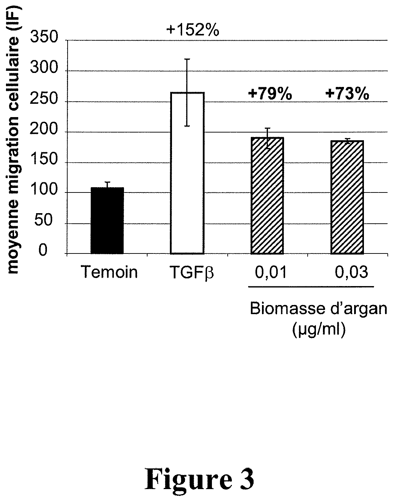

Les résultats sur la migration cellulaire sont rassemblés sur la figure 3 qui illustre l'effet de biomasse d'argan préparée selon l'exemple 2a sur la migration de kératinocytes HaCaT.

Ils montrent que la biomasse d'argan préparée selon l'exemple 2a, à 0.01 ou à 0.03 µg/mL, stimule la migration des kératinocytes HaCaT de 79% et de 73% respectivement.

**Revendications**

1. Utilisation cosmétique d'une préparation issue d'une culture in vitro de cellules dédifférenciées non élicitées d'Arganier, dans le traitement non thérapeutique du vieillissement cutané.

2. Utilisation selon la revendication 1, **caractérisée en ce que** ladite préparation se présente sous la forme de suspension cellulaire, biomasse, biomasse broyée, surnageant de biomasse broyée ou surnageant de culture.

3. Utilisation cosmétique d'une composition cosmétique comprenant à titre de principe actif une préparation issue d'une culture de cellules dédifférenciées non élicitées d'Arganier en association avec un excipient cosmétiquement acceptable, dans le traitement non thérapeutique du vieillissement cutané.

4. Utilisation selon la revendication 3, **caractérisée en ce que** ladite préparation se présente sous la forme de suspension cellulaire, biomasse, biomasse broyée, surnageant de biomasse broyée ou surnageant de culture.

5. Utilisation selon l'une des revendications 3 et 4, **caractérisée en ce que** la quantité de préparation est comprise entre 0,1 et 10 % en poids total de la composition.

6. Procédé de traitement cosmétique du vieillissement cutané, impliquant l'utilisation d'une composition comprenant à titre de principe actif une préparation issue d'une culture de cellules dédifférenciées non élicitées d'Arganier en association avec un excipient cosmétiquement acceptable.

7. Procédé selon la revendication 6, **caractérisé en ce que** ladite préparation se présente sous la forme de suspension cellulaire, biomasse, biomasse broyée, surnageant de biomasse broyée ou surnageant de culture.

8. Procédé selon l'une des revendications 6 et 7, **caractérisé en ce que** la quantité de préparation est comprise entre 0,1 et 10 % en poids total de la composition.

**Patentansprüche**

1. Kosmetische Verwendung einer Präparation aus einer In-vitro-Kultur von undifferenzierten, nicht von einem Arganbaum elizitierten Zellen, bei der nicht therapeutischen Behandlung der Hautalterung.

2. Verwendung nach Anspruch 1, **dadurch gekennzeichnet, dass** sich die besagte Präparation in Form einer Zellsuspension, Biomasse, zerkleinerten Biomasse, Überstand einer zerkleinerten Biomasse oder Kulturüberstand darstellt.

3. Kosmetische Verwendung einer kosmetischen Zusammensetzung, als Wirkstoff eine Präparation aus einer Kultur von undifferenzierten, nicht von einem Arganbaum elizitierten Zellen in Verbindung mit einem kosmetisch verträglichen Hilfsstoff umfassend, bei der nicht therapeutischen Behandlung der Hautalterung.

4. Verwendung nach Anspruch 3, **dadurch gekennzeichnet, dass** sich die besagte Präparation in Form einer Zellsuspension, Biomasse, zerkleinerten Biomasse, Überstand einer zerkleinerten Biomasse oder Kulturüberstand darstellt.

5. Verwendung nach einem der Ansprüche 3 und 4, **dadurch gekennzeichnet, dass** die Präparationsmenge zwischen 0,1 und 10% im Gesamtgewicht der Zusammensetzung enthalten ist.

6. Verfahren zur kosmetischen Behandlung der Hautalterung, welche die Verwendung einer Zusammensetzung impliziert, die als Wirkstoff eine Präparation aus einer Kultur von undifferenzierten, nicht von einem Arganbaum elizitierten Zellen in Verbindung mit einem kosmetisch verträglichen Hilfsstoff umfasst.

7. Verfahren nach Anspruch 6, **dadurch gekennzeichnet, dass** sich die besagte Präparation in Form einer Zellsuspension, Biomasse, zerkleinerten Biomasse, Überstand einer zerkleinerten Biomasse oder Kulturüberstand darstellt.

8. Verfahren nach einem der Ansprüche 6 und 7, **dadurch gekennzeichnet, dass** die Präparationsmenge zwischen 0,1 und 10% im Gesamtgewicht der Zusammensetzung enthalten ist.

**Claims**

1. Cosmetic use of a preparation obtained from an in vitro dedifferentiated non-elicited argan cell culture, in the non-therapeutic treatment of skin ageing.

2. Use according to claim 1, **characterised in that** said preparation is presented in the form of a cell suspension, biomass, milled biomass, milled biomass supernatant or culture supernatant.

3. Cosmetic use of a cosmetic composition comprising by way of active ingredient a preparation obtained from an in vitro dedifferentiated non-elicited argan cell culture in association with a cosmetically acceptable excipient, in the non-therapeutic treatment of skin ageing.

4. Use according to claim 3, **characterised in that** said preparation is presented in the form of a cell suspension, biomass, milled biomass, milled biomass supernatant or culture supernatant.

5. Use according to one of claims 3 and 4, **characterised in that** the quantity of preparation is between 0.1 and 10% of the total weight of the composition.

6. Method for the cosmetic treatment of skin ageing, involving the use of a composition comprising by way of active ingredient a preparation obtained from an in vitro dedifferentiated non-elicited argan cell culture in association with a cosmetically acceptable excipient.

7. Method according to claim 6, **characterised in that** said preparation is presented in the form of a cell suspension, biomass, milled biomass, milled biomass supernatant or culture supernatant.

8. Method according to one of claims 6 and 7, **characterised in that** the quantity of preparation is between 0.1 and 10% of the total weight of the composition.

**Figure 1**

**Figure 2**

**Figure 3**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 1213025 A
- EP 1213024 A
- EP 1430900 A
- EP 1968536 A
- WO 03077881 A

**Littérature non-brevet citée dans la description**

- **MURASHIGE, T. ; SKOOG, F.** A revised médium for rapid growth and bio assays with tobacco tissue cultures. *Physiol. Plant,* 1962, vol. 15, 473-496
- Formulations and Uses. **E. F. GEORGE ; D. J. M. PUTTOCK ; H. J. GEORGE.** Plant Culture Media. Exegetics Ltd, 1987, vol. 1
- A revised medium for rapid growth and bio assays with tobacco tissue cultures. **MURASHIGE, T. ; SKOOG, F.** Physiol. Plant. 1962, vol. 15, 473-496
- **IGOR POPOV ; GUDRUN LEWIN.** *Methods in enzymology [44,* vol. 300, 437-456
- **MAIBACH I HOWARD.** *Journal of Cosmetic Dermatology,* 2008, vol. 7 (2), 96-100
- **A. VAN DER VLIET.** *Chem Biol Interaction,* 1992, vol. 85, 95-116
- **HIDEAKI NAGASE ; J. FREDERICK WOESSNER.** *J Biol Chem,* 30 Juillet 1999, vol. 274 (31), 21491-21494
- **DAVID LEPPERTD.** *Analytical Biochemistry,* 2004, vol. 328, 166-17